(19) Europäisches Patentamt
European Patent Office
Office européen des brevets

(11) Numéro de publication: 0 354 134 A1

(12) **DEMANDE DE BREVET EUROPEEN**

(21) Numéro de dépôt: 89420259.7

(22) Date de dépôt: 18.07.89

(51) Int. Cl.5: C 07 C 43/23
C 07 C 41/14, C 07 C 41/26

(30) Priorité: 05.08.88 FR 8810812

(43) Date de publication de la demande: 07.02.90 Bulletin 90/06

(84) Etats contractants désignés: AT BE CH DE ES FR GB GR IT LI LU NL SE

(71) Demandeur: RHONE POULENC CHIMIE
25 Quai Paul Doumer
F-92408 Courbevoie Cédex (FR)

(72) Inventeur: Gubelmann, Michel
39, Boulevard des Belges
F-69006 Lyon (FR)

Allandrieu, Christian
8, avenue Salvador Allende
F-69100 Villeurbanne (FR)

(74) Mandataire: Vignally, Noel et al
Rhône-Poulenc Chimie Service Brevets Chimie Centre de Recherches des Carrières B.P. 62
F-69192 Saint-Fons Cédex (FR)

(54) **Procédé de monométhylation de composés phénoliques.**

(57) La présente invention concerne un procédé de monométhylation de composés phénoliques.

Il a été trouvé, et cela constitue l'objet de la présente invention, que le paradiméthoxybenzène constitue un produit permettant de réaliser, sélectivement, la méthylation d'un groupe phénol de composés phénoliques.

EP 0 354 134 A1

## Description

## PROCEDE DE MONOMETHYLATION DE COMPOSES PHENOLIQUES

La présente invention concerne un procédé de monométhylation de composes phénoliques.

On a souvent besoin de pouvoir disposer, en tant que produits intermédiaires utiles pour les synthèses organiques, de composés phénoliques (mono, di ou triphénols), dans lesquels l'une des fonctions phénol est méthylée, c'est-à-dire est remplacée par la fonction méthoxy ($-OCH_3$). La préparation de tels produits intermédiaires, à partir des composes phénoliques, nécessite que l'on solutionne convenablement des réactifs susceptibles de méthyler ladite fonction phénolique et les conditions de réaction desdits réactifs, de façon à obtenir sélectivement, c'est-à-dire sans apparition de produits secondaires non recherchés en trop grandes quantités, les produits visés. Il se trouve par ailleurs que, dans la chimie des composés diphénoliques, on prépare, comme sous-produits, du paradiméthoxybenzène que l'on cherche à valoriser.

Il a été trouvé, et cela constitue l'objet de la présente invention, que le paradiméthoxybenzène constitue un produit permettant de réaliser, sélectivement, la méthylation d'un groupe phénol de composés phénoliques.

Les composés phénoliques qu'il est ainsi possible de méthyler sont les composés de formule :

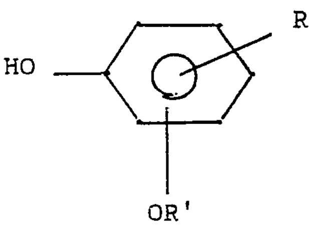

(I)

dans laquelle
- R est choisi parmi H, OH, alkyle, alcoxy, halogène, phényle éventuellement substitué par alkyle, $CF_3$ ;
- R4 est choisi parmi H, alkyle, $C_6H_4OH$ et $C_6H_4O$-alkyle.

Dans la présente demande, le terme "alkyle" signifie alkyle saturé linéaire ou ramifié comportant de 1 à 4 atomes de carbone

Les composés phénoliques de formule (I) les plus intéressants à utiliser sur le plan industriel sont les diphénols, c'est-à-dire les composés, dans lesquels R est H et R′ est H, le groupe OH étant alors situé en position ortho ou para par rapport à l'autre groupe OH.

La réaction réalisée selon l'invention peut s'écrire :

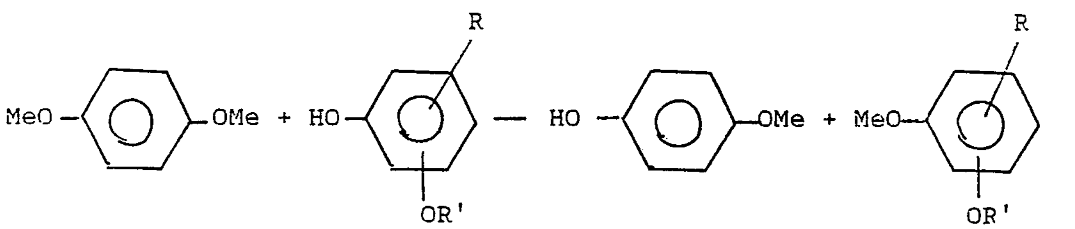

(II)

Me représentant le groupe méthyle ; R et R′ ayant la signification donnée ci-dessus.

La réaction (II) est une réaction équilibrée ; son rendement dans le produit final visé peut donc dépendre des quantités relatives de réactifs mis en jeu. On utilisera avantageusement, notamment sur le plan industriel, les quantités optimales de réactifs en tenant compte de la cinétique de la réaction, de l'équilibre, du coût des produits et également des éventuelles réactions secondaires.

La réaction selon l'invention est réalisée en présence d'un catalyeur qui est un acide quelconque et notamment les acides solides possédant des fonctions acides selon les définitions de lewis ou de Bronsted. Les acides utilisés peuvent dont être liquide ($SO_4H_2$, par exemple) ou solides. Les catalyseurs solides qui constituent les catalyseurs préférés peuvent être définis comme tous solides possédant une capacité échangeuse de cations qui a été partiellement ou complètement échangée par $H^+$ et/ou un acid de Lewis (dérivé d'un élément de valence $\geqq 3$). Parmi ces solides, on peut citer par exemple :
- les argiles qui ont été traitées par un acide fort
- les zéolites qui ont également subi des échanges avec des acides forts ou qui ont été obtenues, comme connu, par décomposition de sels d'ammonium de zéolites ammoniées correspondantes
- des résines du type styrène-divinylbenzène macroéticulées et sulfonées
- des oxydes acides ou amphotères dont l'activité acide peut être avantageusement exaltée grâce à un traitement par un acide
- des hétéropolyacides tels que les acides phosphomolybdiques ou phosphovanadiques, etc.

Les conditions de la réaction dépendent bien évidement d'une par des réactifs et d'autre part des catalyseurs.

Les températures de réaction peuvent se situer entre 80 et 400° C, mais on utilisera de préférence des températures comprises entre 150 et 250° C.

Le milieu de réaction peut être constitué par un liquide inerte aux températures utilisées, c'est le cas notamment, mais de façon non limitative, lorsque l'on met en oeuvre un catalyseur liquide. Lorsque l'on utilise un catalyseur solide, la réaction peut se dérouler de préférence par simple contact entre ledit catalyseur et les réactifs - qui sont alors gazeux - à la température choisie. Bien évidemment, il sera le plus souvent indispensable, compte tenu de la température d'ébullition des réactifs et de la température choisie pour la réaction, d'opérer sous pression.

Les proportions relatives des réactifs à utiliser peuvent être variables pour tenir compte, notamment, comme expliqué ci-dessus que la réaction visée est équilibrée. Cependant, comme dans la présente invention, on vise essentiellement la méthylation d'une seule fonction phénol du composé phénolique, on utilisera de préférence les réactifs (diméthoxybenzène et composé phénolique) en quantités sensiblement équimoléculaires.

Les quantités de catalyseur à utiliser dépendent essentiellement de l'activité dudit catalyseur, activité qui est en relation directe avec la capacité d'échange ionique du catalyseur employé.

Quand on utilise un acide liquide tel que l'acide sulfurique, par exemple dont la capacité d'échange est de 20 méq./g (méq. = milliéquivalent), on utilisera de préférence de 0,05 à 0,5 g d'acide concentré pour 5 à 50 mmol de paradiméthoxybenzène.

Quand on utilisera par exemple un catalyseur solide ayant une capacité d'échange de 0,5 à 1,5 meq./g, on utilisera environ 1 g de ce catalyseur pour 5 à 50 mmol de paradiméthoxybenzène.

La mise en oeuvre de l'invention telle que décrite ci-dessus permet d'obtenir une réaction présentant simultanément un bon taux de transformation des deux réactifs et une sélectivité élevée vis-à-vis du produit final visé, c'est-à-dire du composé phénolique dans lequel l'une des fonctions phénoliques a été méthylée. Le taux de transformation de chacun des réactifs est généralement de l'ordre de 20 à 80 % et la sélectivité en produit final visé est généralement de l'ordre de 60 à 95 %.

Les exemples non limitatifs ci-après illustrent l'invention.

EXEMPLE 1

Dans un tube de verre, on introduit 1 g d'une zéolite commerciale de type faujasite (US-Y de TOYO-SODA), 2,5 g (soit 22,7 mmol) d'hydroquinone (HQ) et 3,13 g (soit 21,7 mmol) de paradiméthoxybenzéne (PDMB). Après avoir été scellé, le tube est introduit dans une gaine métallique qui est placée dans un four à balancement (dit de CARIUS). La température du four est portée à 200° C pendant 2,5 heures.

A la fin de la réaction, l'ensemble tube-gaine est refroidi, le tube est sorti de sa gaine et ouvert. Le catalyseur solide est récupéré par filtration sur verre fritté et lavé avec de l'acétate d'éthyle. Le filtrat et le liquide de lavage sont additionnés d'acétate d'éthyle jusqu'à obtenir un volume de 100 ml. Le mélange liquide obtenu est analysé par chromatographie en phase gazeuse et la nature des produits a été confirmée par spectrométrie de masse.

Le taux de conversion (TT) c'est-à-dire le pourcentage des réactifs de départ consommés a été :
- pour l'hydroquinone (HQ) de 54 %
- pour le paradiméthoxybenzène (PDMB) de 58 %.

La sélectivité de la réaction (RT) définie comme étant la nombre de moles de produit final (paraméthoxyphénol) obtenu par rapport au nombre de moles du réactif de départ transformé est de 77 %.

EXEMPLE 2

On procède comme à l'exemple 1, mais avec une argile acide commerciale (Montmorillonite-KSF de Süd-Chemie, Munich - RFA) comme catalyseur.

TT (HQ) = 53 %
TT (PDMB) = 69 %
RT (PMP) = 65 %

EXEMPLE 3

On procède comme à l'exemple 1, mais avec de la pyrocatéchine (PC) au lieu de l'hydroquinone.

TT (PC) = 34 %
TT (PDMB) = 48 %
RT (OMP) = 53 %
(où : OMP = ortho-méthoxy-phénol)
RT (PMP) = 59 %

EXEMPLE 4

On procède comme à l'exemple 2, mais avec de la pyrocatéchine au lieu de l'hydroquinone.

TT (PC) = 35 %
TT (PDMB) = 59 %
RT (OMP) = 73 %
RT (PMP) = 70 %

EXEMPLE 5

Cet exemple (ainsi que les exemples 6 à 9) illustre la mise en oeuvre du procédé de l'invention en utilisant un catalyseur solide et en opérant à pression atmosphérique.

Dans un réacteur cylindrique en verre de 30 $cm^3$ muni d'une agitation magnétique et d'un réfrigérant, on introduit 3,2 g (23,2 mmol) de PDMB, 2,5 g (22,7 mmol) d'HQ et 1 g d'une argile acide commerciale (TONSIL OPTIMUM FF de Süd-chemie, Munich - RFA). Ce mélange est chauffé à 170° C pendant 3 heures avec une bonne agitation. Le solide acide est récupéré par filtration sur verre fritté et lavages avec de l'acétate d'éthyle. Tous les produits organiques sont dosés par chromatographie en phase gazeuse. Leur nature est confirmée par spectrométrie de masse.

TT (PDMB) = 31 %
TT (HQ) = 38 %
RT (PMP) = 60 %

EXEMPLE 6 A 9

On procède comme dans l'exemple 5 mais en utilisant les conditions suivantes :

. charges : PDMB 0,6 g ; 4,4 mmol
HQ 5,1 g ; 46,4 mmol
catalyseur 0,5 - 1,0 g
. température : 170° C
. durée : 1-3 heures.

Les conditions utilisées (choix du catalyseur - quantité de catalyseur employée - durée de la réaction) et les résultats obtenus sont rassemblés dans le tableau 1.

TABLEAU 1

| Ex. | Solide acide | | Durée (h) | TT PDMB (%) | TT HQ (%) | RT PMP (%) |
|---|---|---|---|---|---|---|
| | nature | masse (g) | | | | |
| 6 | Argile KSF | 1 | 3 | 64 | 45 | 76 |
| 7 | Argile KSF | 1 | 1 | 39 | 27 | 92 |
| 8 | NAFION 117-H | 1 | 1 | 78 | 41 | 59 |
| 9 | idem | 0,5 | 1 | 63 | 28 | 59 |

Le NAFION est un produit, commercialisé par DUPONT DE NEMOURS, qui est un polymère perfluoré de formule générale :

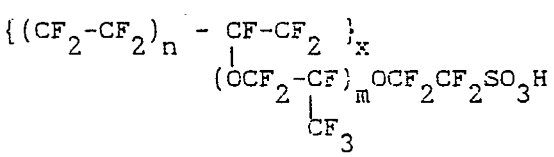

dans laquelle
- n est un nombre compris entre 5 et 13,5,
- m est égal à nombre entier égal à 1,2 ou 3,
- x a une valeur d'environ 1 000.

Ces produits ont des propriétés d'échange ionique acide et le NAFION 117-H a une capacité d'échange de 0,9 méq./g de solide sec.

EXEMPLE 10 ET 11

Ces exemples 10 et 11 illustrent la mise en oeuvre du procédé selon l'invention en utilisant un catalyseur solide en opérant à pression atmosphérique et en présence d'un cosolvant.

Le mode de mise en oeuvre est identique à celui décrit dans l'exemple 5 mais en utilisant :

. comme charges : PDMB 2,6 g (18,8 mmol)
HQ 2,2 g (20 mmol)
catalyseur 1 g d'argile acide
(TONSIL OPTIMUM FF DE Süd-chemie)
cosolvant 1,5 g
. comme température : 200° C
. une durée de réaction de 2 heures.

Dans un essai comparatif réalisé dans les mêmes conditions, avec les mêmes réactifs mais en absence de

catalyseur et de cosolvant, on a obtenu les résultats suivants :
TT (PDMB) = 2 %
TT (HQ) = 3 %
RT (PMP) = 0 %

Les résultats obtenus en utilisant comme solvant, soit du mésitylène, soit de l'ortho-dichloro-benzène (ODCB) sont rassemblés dans le tableau 2.

TABLEAU 2

| Exemple | Cosolvant | TT PDMB (%) | TT HQ (%) | RT PMP (%) |
|---|---|---|---|---|
| 10 | mésitylène | 30 | 43 | 70 |
| 11 | ODCB | 30 | 43 | 70 |

EXEMPLE 12 A 14

Ces exemples illustrent l'utilisation d'un catalyseur liquide qui est l'acide sulfurique concentré.

On procède comme dans les exemples 10 et 11 mais en remplaçant l'argile par 0,15 g d'acide sulfurique concentré (98 %) ; on utilise également un cosolvant que est soit du mésitylène (exemple 12), soit l'éther diméthylique du diéthylèneglycol (exemple 13 diglyme), soit de l'ortho-dichloro-benzène (ODCB).

Les résultats sont rassemblés dans le tableau 3.

TABLEAU 3

| Exemple | Cosolvant | TT PDMB (%) | TT HQ (%) | RT PMP (%) |
|---|---|---|---|---|
| 12 | mesitylène | 45 | 62 | 70 |
| 13 | diglyme | 27 | 73 | < 100 |
| 14 | ODCB | 58 | 74 | 57 |

EXEMPLE 15-16

On peut également utiliser un catalyseur acide liquide sans addition de cosolvant ; c'est ce qui est illustré dans les exemples 15 et 16.

On opère comme dans l'exemple 5 en utilisant :
. comme charges : PDMB 3,2 g
HQ 2,5 g
catalyseur liquide 0,1 g
. comme température : 170° C
. comme durée : 3 heures.

Les résultats obtenus, avec $SO_4H_2$ à 98 % ou $F_3CSO_3H$, sont rassemblés dans le tableau 4.

TABLEAU 4

| Exemple | Acide liquide | TT PDMB (%) | TT HQ (%) | RT PMP (%) |
|---|---|---|---|---|
| 15 | $H_2SO_4$ 98 % | 38 | 53 | 63 |
| 16 | $F_3CSO_3H$ | 78 | 79 | 27 |

**Revendications**

1. Procédé pour réaliser la monométhylation de composés phénoliques de formule :

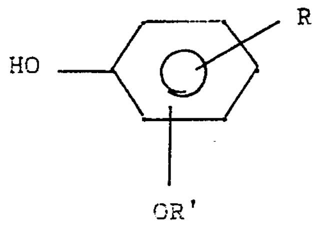

dans laquelle- R est choisi parmi H, OH, alkyle, alcoxy, halogène, phényle éventuellement substitué par un radical alkyle, $CF_3$, et
- R′ est choisi parmi H, alkyl, $C_6H_4OH$ et $C_6H_4O$-alkyle,
ledit procédé étant caractérisé en ce qu'on utilise comme agent de méthylation le paradiméthoxybenzène en présence d'un catalyseur acide.

2. Procédé selon la revendication 1, caractérisé en ce que l'on utilise comme catalyseur un acide liquide tel que l'acide sulfurique.

3. Procédé selon la revendication 1, caractérisé en ce que le catalyseur utilisé est un solide possédant une capacité d'échange cationique.

4. Procédé selon l'une des revendications 1 à 3, caractérisé en ce que l'on utilise de préférence des quantités sensiblement équimoléculaires desdits composés phénoliques de formule (I) et de paradiméthoxybenzène et que l'on opère à une température comprise entre environ 80 et environ 400° C et de préférence de 150 à 250° C.

5. Procédé selon l'une des revendications 1 à 4, caractérisé en ce que ledit composé phénolique de formula (I) est choisi parmi les diphénols, c'est-à-dire les composés dans lesquels R et R′ sont H et dans lesquels OR′ est en position ortho ou para par rapport à OH.

6. Procédé selon l'une des revendications 2, 4 et 5 caractérisé en ce que l'on utilise de préférence de 0,05 à 0,5 g d'acide concentré pour 5 à 50 mmol de paradiméthoxybenzène.

7. Procédé selon l'une des revendications 3, 4 et 5 caractérisé en ce que l'on utilise environ 1 g de catalyseur solide ayant une capacité d'échange de 0,5 à 1,5 milliéquivalent/g, pour 5 à 50 mmol de paradiméthoxybenzène.

Office européen des brevets

# RAPPORT DE RECHERCHE EUROPEENNE

Numero de la demande

EP 89 42 0259

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | US-A-2 697 732 (J.M. MAVITY)<br>* En entier *<br>--- | 1-7 | C 07 C 43/23<br>C 07 C 41/14<br>C 07 C 41/26 |
| Y | EP-A-0 013 924 (BAYER)<br>* Revendications; exemples 2,3 *<br>--- | 1-7 | |
| A | CHEMICAL ABSTRACTS, vol. 106, no. 19, 11 mai 1987, page 617, résumé no. 155658b, Columbus, Ohio, US; P. BELTRAME et al.: "Methyl transfer from anisole to phenol on HY zeolite", & GAZZ. CHIM. ITAL. 1986, 116(8), 473-4<br>----- | 1-7 | |

| DOMAINES TECHNIQUES RECHERCHES (Int. Cl.5) |
|---|
| C 07 C 41/00<br>C 07 C 43/00 |

Le présent rapport a été établi pour toutes les revendications

| Lieu de la recherche | Date d'achèvement de la recherche | Examinateur |
|---|---|---|
| LA HAYE | 07-11-1989 | WRIGHT M.W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)